(19) Europäisches Patentamt
European Patent Office
Office européen des brevets

(11) Publication number: **0 309 724 B1**

(12) **EUROPEAN PATENT SPECIFICATION**

(45) Date of publication of patent specification: **17.06.92**

(51) Int. Cl.5: **C07C 69/63**, C07C 67/307

(21) Application number: **88113585.9**

(22) Date of filing: **22.08.88**

(54) **Improved method for the preparation of dialkyl dichlorosuccinates.**

(30) Priority: **10.01.87 US 101454**

(43) Date of publication of application:
**05.04.89 Bulletin 89/14**

(45) Publication of the grant of the patent:
**17.06.92 Bulletin 92/25**

(84) Designated Contracting States:
**AT BE CH DE ES FR GB GR IT LI LU NL SE**

(56) References cited:
**US-A- 2 527 345**
**US-A- 4 675 432**

**CHEMICAL ABSTRACTS, vol. 75, no. 18, November 1, 1971, Columbus, Ohio, USA; SAIKAWA, YOSHIO, "Dialkyl-D,L-dichlorosuccinates", page 192, column 1, abstract no. 117979r**

(73) Proprietor: **AMERICAN CYANAMID COMPANY**
**1937 West Main Street P.O. Box 60**
**Stamford Connecticut 06904-0060(US)**

(72) Inventor: **Cevasco, Albert Anthony**
**21 Kingswood Drive**
**Belle Mead New Jersey 08502(US)**

(74) Representative: **Wächtershäuser, Günter, Dr.**
**Tal 29**
**W-8000 München 2(DE)**

**EP 0 309 724 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel methods for preparing precursors for quinoline-2,3-dicarboxylic acids. These acids are useful intermediates in the preparation of herbicidal pyridine and quinoline imidazolinone herbicidal compounds.

The herbicidal pyridine and quinoline imidazolinone compounds prepared from the present compounds include 2-(4-isopropyl-4-methyl-5-oxo-2-imidazolin-2-yl)quinoline-3-carboxylic acid, and esters and salts thereof and are disclosed in United States Patent 4,638,068. These herbicidal imidazolinyl quinolinecarboxylic acids may be prepared by the procedure described in United States Patent 4,518,780 by cyclization, under basic conditions, with an appropriately substituted 2-carbamoyl quinoline-3-carboxylic acid, that, in turn, is prepared by the reaction of a substituted quinoline-2,3-dicarboxylic acid anhydride and appropriately substituted aminocarboxamide or aminothiocarboxamide. Quinoline-2,3-dicarboxylic acid anhydrides are readily prepared from the diacids by procedures well known in the art. However, the diacids themselves are not readily available.

US-A- 465 6283 describes a method useful for the preparation of quinoline-2,3-dicarboxylic acid and esters thereof by reacting a beta-anilino-alpha,beta-unsaturated ester with an immonium salt (commonly called a Vilsmeier reagent). The beta-anilino-alpha, beta-unsaturated esters are obtained by the reaction of appropriately substituted anilines with keto-esters or dialkyl acetylene dicarboxylates. The overall reaction for the preparation of quinoline-2,3-dicarboxylates is illustrated in Flow Diagram I.

**FLOW DIAGRAM I**

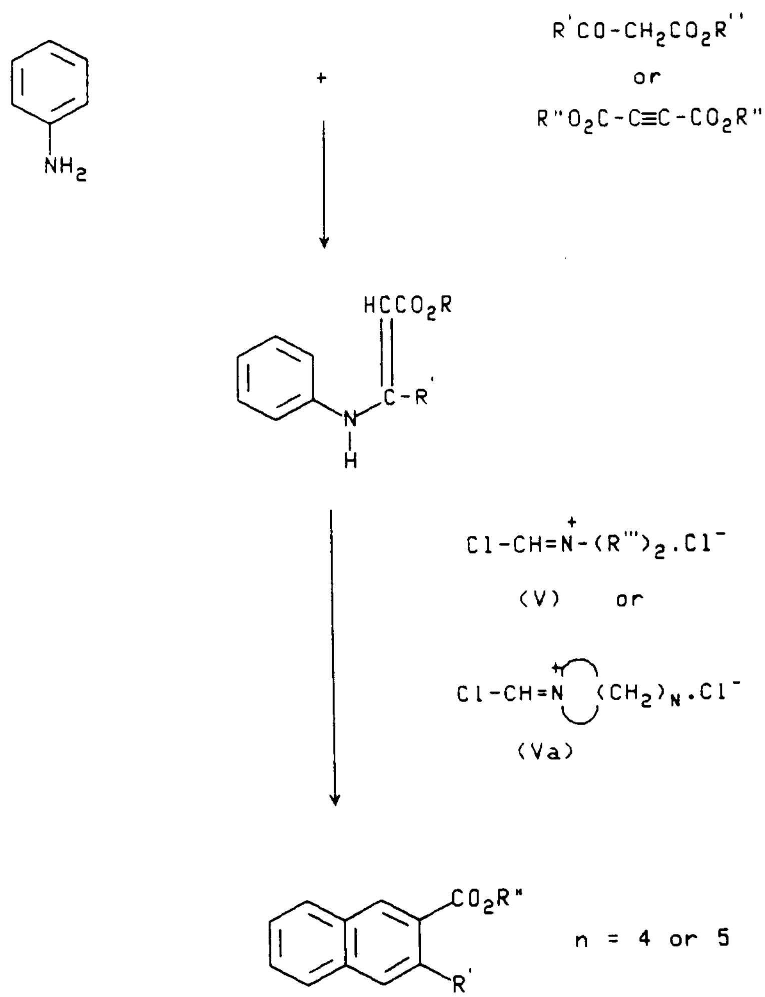


wherein R is $CH_3$ or $CO_2R''$ and $R''$ is $C_1$-$C_4$ alkyl, and $R''''$ is $CH_3$ or $C_1$-$C_4$ alkyl.

Where $R'$ is $CH_3$, the diacid is obtained by concurrent oxidation and hydrolysis of the product under aqueous basic conditions in the presence of nickel peroxide, as described in United States Patent 4,459,409.

Unfortunately, the availability of ketoesters and dialkyl acetylene dicarboxylates, such as diethyloxalacetate and diethyl acetylenedicarboxylate, is limited, thus restricting the quantities of anilinofumarate and quinoline-2,3-dicarboxylic acid, the intermediates required for preparing herbicidal 2-(4-isopropyl-4-methyl-5-oxo-2-imidazolin-2-yl)quinoline-3-carboxylic acid, esters and salts thereof.

United States Patent 4,675,432 describes a method for the preparation of anilinofumarates in which dichlorosuccinates are reacted with aniline in an organic solvent in the presence of aqueous base and a phase transfer catalyst in a temperature in the range of 20 °C to 90 °C for one to 24 hours.

EP-A- 026 2305 describes a method for the preparation of anilinofumarates by the reaction of

dichlorosuccinates with specific amines and the subsequent displacement of the amine with aniline in the presence of an organic acid.

United States Patent 2,527,345 describes a method for the preparation of dialkyl dichlorosuccinates by introducing a stream of chlorine in intimate contact with a liquid dialkyl maleate maintained at a temperature of 25 °C to 125 °C, at a sufficiently high rate to permit a portion of the chlorine gas to pass through the reaction mixture unreacted, in the presence of a chlorination catalyst such as ferric chloride, phosphorus pentoxide or sulfur monochloride.

Japanese Patent Application 71,21,564 describes an improved method for the preparation of dialkyl dichlorosuccinates by adding chlorine to a dialkyl maleate in a chlorinated hydrocarbon solvent in the presence of an alcohol, the reactions being, in general, conducted at low temperatures and in the dark.

It is an object of the present invention to provide an improved method for the preparation of dialkyl dichlorosuccinates which results in increased productivity and shorter reaction times.

## SUMMARY OF THE INVENTION

The present invention provides an improved method for the preparation of dialkyl dichlorosuccinates resulting in significantly increased productivity and reduction in reaction times. In the process of the present invention, dialkylmaleate is reacted with chlorine, in the absence of a solvent, but in the presence of a catalytic quantity of an alcohol, at a temperature range of about 20 °C to about 75 °C, for a sufficient period of time to complete the reaction.

The improved process of this invention provides yields of about 80% to 90% pure dialkyl dichlorosuccinates directly, without the use of a solvent. The dialkyl dichlorosuccinates obtained by the method of this invention may be reacted directly with aniline and a minimum of two molar equivalents of aqueous base in the presence of a phase transfer catalyst to yield anilinofumarates by the procedures described in United States Patent 4,675,432, without further purification.

Additionally, since the method of this invention does not employ a chlorinated hydrocarbon solvent, it reduces handling, effluent and processing steps associated with solvent recovery.

## DETAILED DESCRIPTION OF THE INVENTION

The present invention provides an improved method for the preparation of dialkyl dichlorosuccinates, said method comprising reacting a dialkyl maleate of formula I

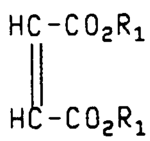

(I)

wherein R is $C_1$-$C_4$ alkyl with 1.0 to 1.5 molar equivalents of chlorine in the presence of 2.5% to 15% of a $C_1$-$C_4$ alkyl alcohol at a temperature range of about 20 °C to 75 °C, preferably 25 °C to 60 °C, for one to five hours.

In accordance with the method of this invention, chlorine gas (150.0 g, 2.12 moles) uniformly is added over two hours to a diethyl maleate (350.0 g, 98% purity, 2.0 moles) and anhydrous ethyl alcohol (9.0 g, 0.2 mole). The temperature of the reaction solution is maintained at 20 °C to 35 °C with external cooling. The solution is stirred for an additional two hours at 25 °C to 40 °C, followed by a nitrogen sparge for one hour at 40 °C to 50 °C to remove excess chlorine to yield 492 g (90%) of diethyl dichlorosuccinate (88.5% pure).

The invention is further illustrated by the following non-limiting examples.

## EXAMPLES 1-8

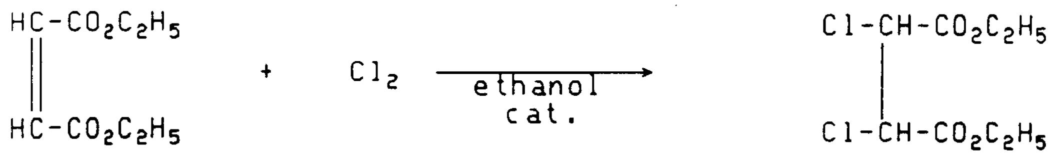


Chlorine gas is added uniformly at various rates to diethyl maleate containing 10 to 15 mol percent of an ethanol, and the resulting reaction mixture is allowed to stir for various periods of time. The resulting mixture is sparged with nitrogen to give the yields of diethyl dichlorosuccinate listed in Table I below.

TABLE I

| Example | Diethyl maleate g | Purity | mols | Ethanol mols | Chlorine mols | Chlorine Feed rate g/min | Time Temp °C | Time Chlorine addition min | Hold hr | % Yield diethyl dichloro-succinate |
|---|---|---|---|---|---|---|---|---|---|---|
| 1 | 17.5 | 99.0 | 0.1 | 0.015 | .10 | 0.16 | 20 - 32 | 50 | 3.0 | 87.3 |
| 2 | 35.0 | 99.0 | 0.201 | 0.02 | .22 | 0.12 | 25 - 32 | 125 | 2.0 | 89.1 |
| 3 | 35.0 | 99.0 | 2.01 | 0.2 | 2.12 | 1.25 | 22 - 32 | 120 | 2.0 | 89.7 |
| 4 | 400.0 | 96.0 | 2.23 | 0.2 | 2.42 | 1.63 | 23 - 37 | 105 | 2.5 | |
| 5 | 225.0 | 78.4 | 1.02 | 0.13 | 1.19 | 0.94 | 23 - 32 | 90 | 1.75 | 94.1 |
| 6 | 1156.4 | 95.7 | 6.428 | 0.64 | 6.79 | 3.21 | 25 - 40 | 150 | 2.25 | - |
| 7 | 483.8 | 94.9 | 2.67 | 0.27 | 2.95 | 1.61 | 25 - 34 | 130 | 2.0 | 92.5 |
| 8 | 513.3 | 96.0 | 2.86 | 0.3 | 3.8 | 1.92 | 15 - 35 | 140 | 16.0 | 88.1 |

## Claims

1. A method for the preparation of dialkyl dichlorosuccinates, said method consisting of reacting dialkyl maleate with chlorine in the presence of catalytic amount of an alcohol at a temperature range of from

20 ° C to 75 ° C for sufficient period of time to complete said reaction.

2. A method according to claim 1, wherein said chlorine is present at 1.0 to 1.5 molar equivalents.

3. A method according to claim 2, wherein said alcohol is $C_1$ - $C_4$ alkyl alcohol.

4. A method according to claim 3, wherein said time is one to five hours.

5. A method according to claim 4, wherein said alcohol is present in a range of from 2.5 to 15 mole % relative to the dialkyl maleate.

6. A method according to claim 5, wherein said alcohol is ethanol.

7. A method according to claim 6, wherein said temperature is 25 ° C to 60 ° C.

**Revendications**

1. Procédé de préparation de dichlorosuccinates de dialkyle, selon lequel on fait réagir un maléate de dialkyle avec du chlore en présence d'une quantité catalytique d'un alcool à une température de 20 ° C à 75 ° C pendant une période suffisante pour achever la réaction.

2. Procédé selon la revendication 1, dans lequel le chlore est présent à raison de 1,0 à 1,5 équivalents molaires.

3. Procédé selon la revendication 2, dans lequel l'alcool est un alcool alkylique en $C_1$-$C_4$.

4. Procédé selon la revendication 3, dans lequel ladite période est de une à cinq heures.

5. Procédé selon la revendication 4, dans lequel l'alcool est présent selon une quantité de 2,5 à 15 moles % par rapport au maléate de dialkyle.

6. Procédé selon la revendication 5, dans lequel l'alcool est l'éthanol.

7. Procédé selon la revendication 6, dans lequel la température est de 25 ° C à 60 ° C.

**Patentansprüche**

1. Verfahren zur Herstellung von Dialkyldichlorsuccinaten, umfassend die Umsetzung von Dialkylmaleat mit Chlor in Gegenwart von katalytischen Mengen eines Alkohols bei einer Temperatur im Bereich von 20 ° C bis 75 ° C während einer ausreichenden Zeitspanne, um die Reaktion zu vervollständigen.

2. Verfahren gemäß Anspruch 1, wobei das Chlor mit 1,0 bis 1,5 molaren Äqivalenten anwesend ist.

3. Verfahren gemäß Anspruch 2, wobei der Alkohol ein $C_1$-$C_4$-Alkylalkohol ist.

4. Verfahren gemäß Anspruch 3, wobei die Zeitspanne 1 bis 5 Stunden beträgt.

5. Verfahren gemäß Anspruch 4, wobei der Alkohol in einem Bereich von 2,5 bis 15 Mol.%, relativ zu den Dialkylmaleat, anwesend ist.

6. Verfahren gemäß Anspruch 5, wobei der Alkohol Ethanol ist.

7. Verfahren gemäß Anspruch 6, wobei die Temperatur 25 ° C bis 60 ° C beträgt.